# EUROPEAN PATENT SPECIFICATION

(11) **EP 2 734 842 B1**
(45) Date of publication and mention of the grant of the patent: **20.01.2016**
(21) Application number: 12738592.0
(22) Date of filing: 19.07.2012
(51) Int. Cl.: G01N 33/543, G01N 33/558

(54) **APPARATUS AND METHOD FOR LATERAL FLOW AFFINITY ASSAYS**
VORRICHTUNG UND VERFAHREN FÜR AFFINITÄTS QUERFLUSSASSAY
APPAREIL ET PROCÉDÉ UTILISÉS POUR RÉALISER DES DOSAGES D'AFFINITÉ

(30) Priority: 19.07.2011 GB 201112395
(43) Date of publication of application: 28.05.2014
(73) Proprietor: The Bio Nano Centre Limited, London N1C 4AG (GB)
(72) Inventor: CASS, Anthony Edward George, London NW1 3BT (GB); CELAYA SANFIZ, Almudena, London NW1 3BT (GB); REHAK, Marian, London NW1 3BT (GB)
(74) Representative: Clarke, Lionel Paul
(86) International application number: PCT/GB2012/051733
(87) International publication number: WO 2013/011323

(56) References cited:
- US-A1- 2003 077 642
- US-A1- 2005 170 405

## Description

### Background

Affinity assays are widely used in medical, food, and environmental research and are based on the principle of a reagent binding to a target analyte. The binding interaction is detected through some physico-chemical change, the magnitude of which is proportional to the analyte concentration. Many different reagents have been described including, but not limited to, antibodies, other proteins, nucleic acids, and synthetic receptors.

Antibodies are commonly used as the reagent and many different assay formats using antibodies have been described. These are collectively referred to as immunoassays. One type of immunoassay which is particularly suitable for point of care tests (POCTs) is the lateral flow (LF) immunoassay (also referred to as an immunochromatographic assay).

LF immunoassays are a well-established and robust technology for detection of antigens. They are adapted to operate along a single axis to suit a test strip format and typically employ a sandwich (also referred to as 2-site, reagent excess or non-competitive) format. In this format one of two antibodies is labelled, typically with coloured particles, and dried as a soluble preparation on a LF membrane (typically a hydrophobic nitrocellulose or cellulose acetate membrane). This first antibody dissolves in the sample, whilst a second antibody is fixed to the LF membrane some short distance from the first antibody. Sample solution carries the first antibody to the second through capillary flow and the immune complex that forms during this flow is captured by the second antibody forming a visible line. Excess labelled first antibody is carried past this 'test line' to react at a control line. This provides a qualitative read-out.

More recently the format has been modified such that the second antibody, labelled with biotin, is also soluble and the sandwich complex is formed in solution during the LF before being captured at a streptavidin line. US2010/0267166 describes a device for detecting an analyte, comprising a labelled conjugate comprising a binding member reacted with a first epitope of the analyte and a label, and a biotinylated capture component having a site reactive with a second epitope of the analyte.

It would be advantageous to adapt this assay format further, in order to increase its sensitivity and provide a quantitative numerical readout of the amount of immune complex at the second line, whilst retaining the essential simplicity and robustness of the LF format.

### Summary of the Invention

According to a first aspect of the invention, apparatus for the quantitative analysis of an analyte in a sample comprises:
(i) solid phase; and
(ii) a detector,
wherein the surface of the solid phase comprises:
(a) a first position for sample application; and
(b) a second position distant from the first position,
wherein a first molecule that binds to the analyte and is capable of releasing a detectable species is either deposited on the solid phase or is added to the sample prior to application to the solid phase, and
wherein a second molecule that binds to the analyte is immobilised at the second position, and
wherein an enzyme is immobilised, co-located with the immobilised molecule at the second position, and
wherein the detector is located in close proximity to the immobilised molecule at the second position.

According to a second aspect of the invention, a method for quantitative detection of target molecules in a substrate-containing sample comprises:
a) dissolving a first soluble labelled binding molecule that is capable of releasing a detectable species and a second soluble labelled binding molecule in the sample to form a complex;
b) applying the complex formed in step a) to a solid phase, wherein the surface of the solid phase comprises:
   i) a first position for application of the complex; and
   ii) a second position, distant from the first position
   wherein a molecule that binds to the second labelled binding molecule is immobilised at the second position, wherein an enzyme is immobilised co-located with the immobilised molecule at the second position, and wherein a detector is located in close proximity to the immobilised molecule at the second position; and
c) detecting the detectable species at the detector,
wherein detection of said species is proportional to the target molecule content of the sample.

According to a third aspect of the invention, the apparatus described in the first aspect of the invention is used in the method for quantitative detection of target molecules in a substrate-containing sample according to the second aspect of the invention.

### Description of the Drawings

Figure 1 is a schematic representation of the amplified electrochemical lateral flow (LF) test strip;
Figure 2 is a schematic representation of the amplified electrochemical reaction;
Figure 3 is a graphical representation of the pH change as a function of the enzymatic reaction between urease and urea in a 2mM Tris/HCl buffer solution;
Figure 4 is a graphical representation of the pH change as a function of the enzymatic reaction between pyrophosphate and pyrophosphatase in a solution with a low buffering capacity (2mM Tris/HCl buffer with addition of 2mM MgCl₂);
Figure 5 is a graph showing the effect of increasing the local pH on release of a first encapsulated label;
Figure 6 shows a similar effect to Figure 5 but using potassium ferricyanate as the first label rather than ferrocene carboxylic acid; and
Figure 7 shows the production of current as a function of ferrocene carboxylic acid release from polymeric bead at different time points.

### Detailed Description of the Invention

The present invention addresses the aims of increasing the sensitivity of binding assays and is described in detail with reference to the lateral flow (LF) immunoassay. The invention provides a quantitative numerical readout of the amount of immune complex present in a sample, whilst retaining the simplicity and robustness of the assay format, by replacing the visual estimation of immune complex formation with an electrochemical measurement.

This is achieved, for example, by labeling one of two binding molecules, which may be antibodies, with particles that function as a source of a redox reagent that is detected at the test line. However, other approaches are also effective and these include, but are not limited to: encapsulated surface enhanced resonance Raman scattering (SERRS) active dyes; encapsulated mixture of a fluorescent molecule and a quencher; and encapsulated luciferase substrate to produce light emission at the test line. Therefore, although the invention is illustrated with respect to lateral flow assays, the scope of the invention is not limited to either the lateral flow assay format or to the antibody-antigen interaction detection.

According to a first aspect, the invention provides an apparatus for the quantitative analysis of an analyte in a sample, comprising:
(i) solid phase; and
(ii) a detector,
wherein the surface of the solid phase comprises:
(a) a first position for sample application; and
(b) a second position, distant from the first position,
wherein a first molecule that binds to the analyte and is capable of releasing a detectable species is either deposited on the solid phase or is added to the sample prior to application to the solid phase, and
wherein a second molecule that binds to the analyte is immobilised at the second position, and
wherein an enzyme is immobilised, co-located with the immobilised molecule at the second position, and
wherein the detector is located in close proximity to the immobilised molecule at the second position.

As used herein, the term "distant from" means that the second position is not immediately adjacent to the first position. Preferably, the second position is located downstream from the first position in the direct of flow.

As used herein, the term "close proximity" means that the detector must be positioned sufficiently close to the immobilized molecule at the second position to be able to detect any change in current or charge passed at said second position. Preferably, the detector is located as close as possible to the immobilized molecule at the second position.

The solid phase may be any suitable solid material or membrane, such as porous and/or non-porous surfaces such as silicon, silicon oxide, metallic and metal-coated surfaces, polymeric and polysaccharide surfaces. Preferably, the solid phase is a lateral flow (LF) membrane, preferably comprising chromatogenic media, which may be polymeric or cellulosic, such as hydrophobic nitrocellulose or cellulose acetate.

According to a second aspect, the present invention also provides a method for quantitative detection of target molecules in a substrate-containing sample. The method comprises the following steps:
a) dissolving a first soluble labelled binding molecule that is capable of releasing a detectable species and a second soluble labelled binding molecule in the sample to form a complex;
b) applying the complex formed in step a) to a solid phase, wherein the surface of the solid phase comprises:
   i) a first position for application of the complex; and
   ii) a second position distant from the first position
   wherein a molecule that binds to the second labelled binding molecule is immobilised at the second position, wherein an enzyme is immobilised co-located with the immobilised molecule at the second position, and wherein a detector is located in close proximity to the immobilised molecule at the second position; and
c) detecting the detectable species at the detector,
wherein detection of said species is proportional to the target molecule content of the sample.

Preferably, the target molecule is an antigen and the first and second labelled binding molecules are antibodies. In such an embodiment, the complex formed is an immune complex.

Step (c) may take place as the complex is transported to the second position. Such transportation may be by means of capillary action, microfluidics or electrophoretic migration.

The apparatus described in the first aspect of the invention can be used in the method for quantitative detection of target molecules in a substrate-containing sample according to the second aspect of the invention.

The apparatus and method of the invention are exemplified by the combinations of enzyme and substrate shown in Table 1. However, many other combinations are also within the scope of the invention and would be apparent to one skilled in the art of enzymology.

**Table 1**

| **Enzyme** | **Substrate** |
|---|---|
| Urease | Urea |
| Glucose Oxidase | Glucose |
| Pyrophosphatase | Pyrophosphate |
| Alkaline phosphatase | Phenylphosphate |
| β-lactamase | Penicillin |

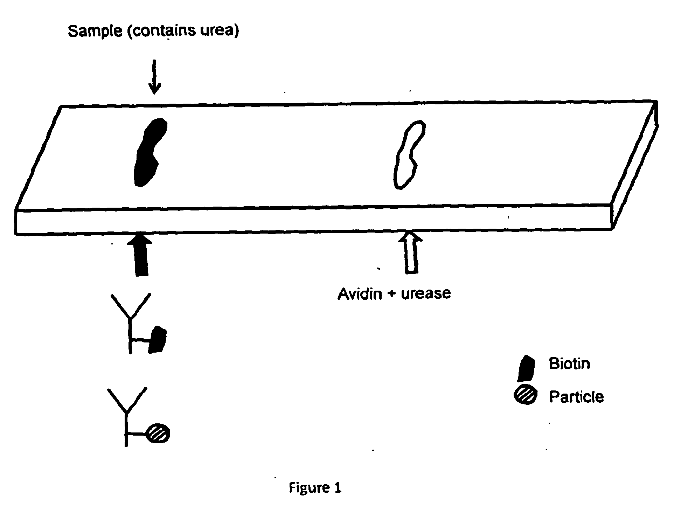

The method utilises the presence of substrate in the sample to change the local pH at the test line through the co-deposition of a corresponding enzyme immobilized at the second position. As would be understood by a person skilled in the art, the term "corresponding enzyme" to refer to an entity that catalysis the chemical reaction with a given substrate. Examples of compositions of the immune complexes that are applied to the solid phase membrane and the test line (i.e. the second position) are illustrated schematically in Figure 1.

The first label that is capable of releasing a detectable species is preferably a redox probe, and is preferably encapsulated in a polymer. The polymer may be an enteric coating material which dissolves when subjected to a pH change, typically under alkaline conditions, or alternatively it may be a 'smart' stimuli responsive polymer that swells in response to a pH change.

Suitable enteric polymers include polyvinyl acetate phthalate, hydroxypropyl methylcellulose phthalate, methacrylic acid, cellulose acetate trimellitate, carboxymethyl ethylcellulose and hydroxypropyl methylcellulose acetate succinate.

Suitable pH-responsive 'smart' polymers include poly(propylacrylic acid) and chitosan.

Suitable redox probes undergo fast, preferably diffusion-limited, electron exchange at the detector and include complexes of iron, osmium, copper and ruthenium, as well as organic molecules such as flavins and dyes. Specific examples include but are not limited to: ferrocene and its derivatives; iron complexes such as hexacyanide; ruthenium complexes such as hexamine; osmium complexes as tris(bipyridyl); thiazine dyes; phenazine dyes; riboflavin and its derivatives; and tetrathiafulvalene and its derivatives. Additional examples will be apparent to those skilled in the art.

The second binding molecule is preferably labelled with biotin and the molecule that binds to it, and which is immobilised at the second position, is preferably avidin or streptavidin.

The detector located in close proximity to the immobilised molecule at the second position is preferably positioned below the test line (i.e. at or underneath the second position on the LF membrane). The detector is preferably an electrode, and preferably a screen printed electrode. The skilled person will be familiar with the term "screen-printed electrode". However, for the avoidance of doubt, this is defined as a conducting carbon ink or metal paste film deposited on an inert support, such as PVC, ceramic, and alumina or polyester, and incorporating reference and counter electrodes. The electrode is preferably poised at a potential where there is a diffusion-limited reduction of the redox probe and a minimal background current from the sample. The detector must be positioned in sufficiently close proximity to the immobilized molecule at the second position (i.e. the test line) to be able to detect the change in current or charge passed.

The apparatus and method of the invention may be used for the *in vitro* quantitative analysis of many analytes including antigens, antibodies, other proteins and the products of nucleic acid amplification tests. The test sample may be selected from the following non-limiting group of body samples obtained from a subject or patient: urine; saliva; serum; plasma; whole blood; faeces; and exudates (e.g. from wounds or lesions). Alternatively, the sample may be a non-clinical material, such as soil, air, water or food matter.

The apparatus and method of the invention can be used as a tool to aid diagnosis and patient management. For example, the assay can be used to identify, confirm, or rule out disease in symptomatic patients, or to accurately prescribe therapeutic drugs and to monitor treatment, for example to monitor blood sugar levels in diabetic patients or to determine pregnancy. Other uses also include in epidemiology, where the rapid assay can be used to detect and monitor the incidence or prevalence of disease for targeting and evaluating health programs, as well as in screening to determine the prevalence of disease in asymptomatic individuals.

The terms 'subject' and 'patient' are used interchangeably herein and refer to a mammal including a non-primate (e.g. a cow, pig, horse, dog, cat, rat and mouse) and a primate (e.g. a monkey and human), and preferably a human.

By way of example, we illustrate an embodiment of the invention as a lateral flow immunoassay wherein the first and second binding molecules are antibodies, the target analyte is an antigen and the solid phase is a lateral flow membrane.

Upon addition of the substrate-containing sample, the two antibodies dissolve and form the immune complex which is carried by lateral flow to the test line. The immune complex travels with the liquid front and is captured at the test line by the reaction between the biotin component of the immune complex and avidin/streptavidin present in the test line. Once the substrate arrives at the test line there is a local change in pH due to the conversion of the substrate within the test sample. This may be a change to a more acidic environment (i.e. a decrease in pH) to a more alkaline environment (i.e. an increase in pH) depending upon the specific enzyme used.

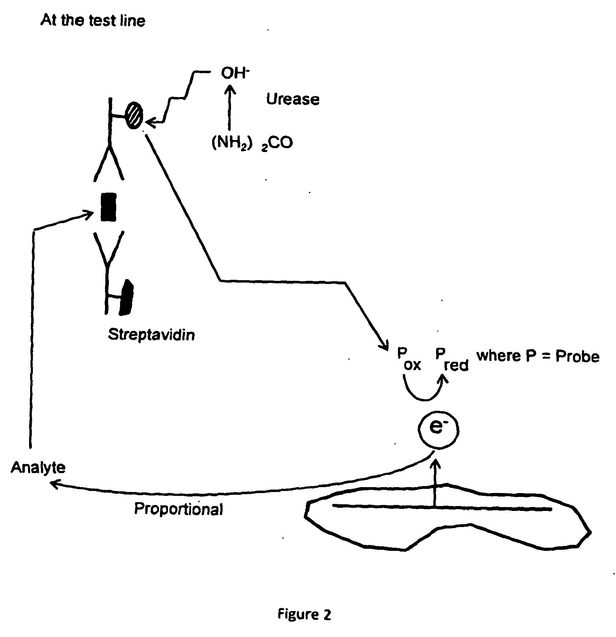

The amplified electrochemical reaction that takes place at the test line is illustrated schematically in Figure 2.

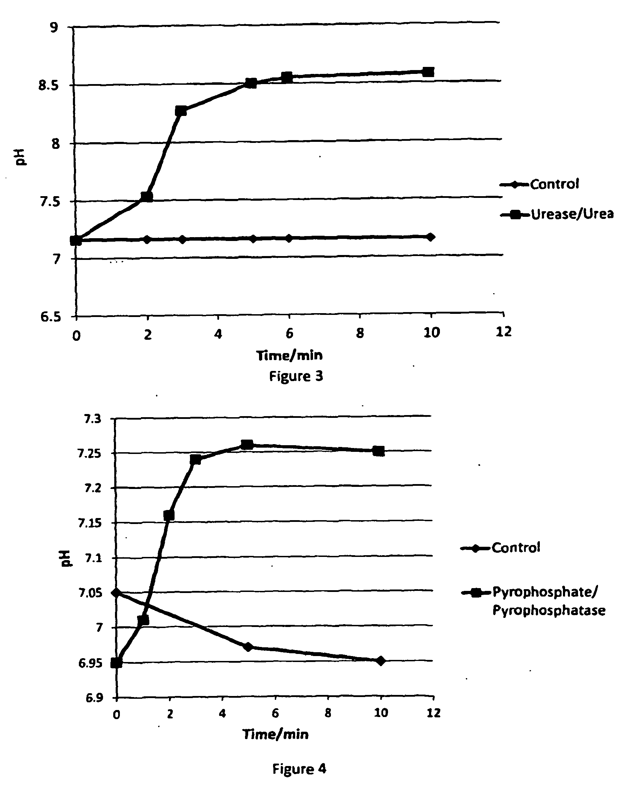

In one embodiment, the substrate-containing sample is preferably urine and the corresponding enzyme is therefore preferably urease. The concentration of urea in urine is around 10-20 times the Km value for urease; therefore the enzyme will be running at its maximal rate. The change in pH results in the redox probe undergoing reduction and releasing the redox species, which is detected from the current (or charge passed) at the underlying electrode. The current resulting from the released redox species is proportional to the antigen content of the sample. Therefore, by measuring the current (or charge passed) at the electrode, the antigen content of the sample can be determined quantitatively. Figure 3 illustrates the pH change as a function of the enzymatic reaction between urease and urea in a solution with a low buffering capacity (2mM Tris/HCl buffer).

Alternatively, the substrate-containing sample may be pyrophosphate and the corresponding enzyme is therefore preferably pyrophosphatase. Figure 4 illustrates the pH change as a function of the enzymatic reaction between pyrophosphate and pyrophosphatase in a solution with a low buffering capacity (2mM Tris/HCl buffer with addition of 2mM MgCl₂).

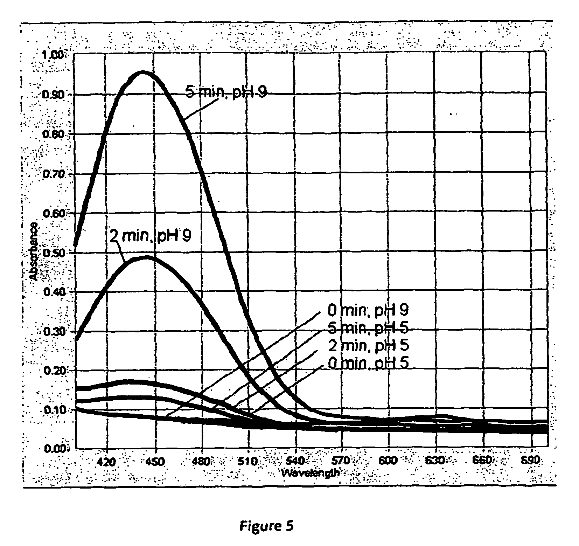

The effect of increasing the local pH on release of the first encapsulated label (a redox probe, specifically a ferrocene derivative) is illustrated in Figure 5. This graph shows the spectrophotometric determination of ferrocene carboxylic acid release from polymeric beads as a function of time, at an alkaline pH of 9. As can be seen from the control values, when the pH is maintained at a mildly acidic pH of 5 the polymer does not dissolve and there is no increase in absorbance.

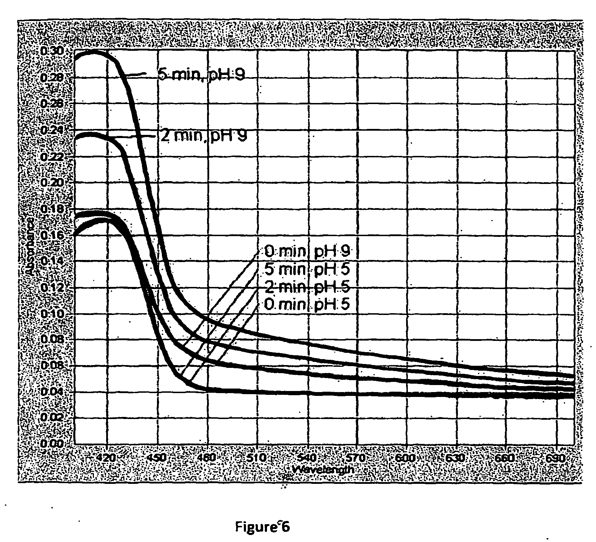

Figure 6 illustrates a similar effect, however in this experiment the redox probe is potassium ferricyanate. The graph shows the spectrophotometric determination of release of the encapsulated probe from polymeric beads as a function of time, at an alkaline pH of 9. As can be seen from the control values, when the pH is maintained at a mildly acidic pH of 5 the polymer does not dissolve and there is no increase in absorbance.

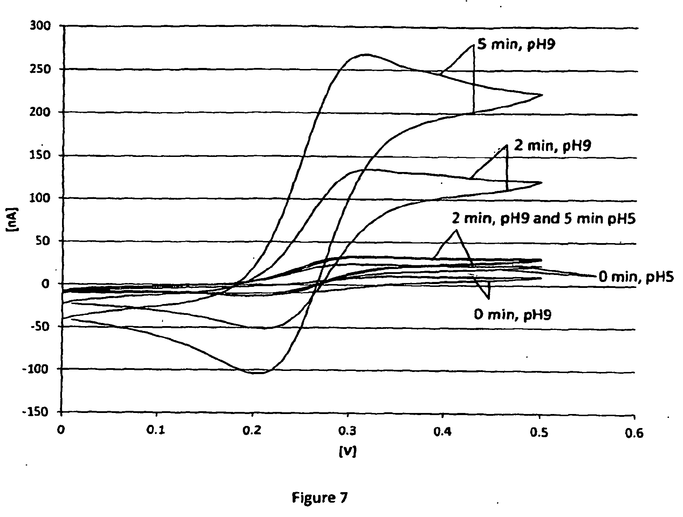

Figure 7 illustrates the production of current as a function of the ferrocene carboxylic acid release from the polymeric beads at different time points and at an alkaline test pH of 9 and a mildly acidic control pH of 5. The graph shows that at pH 9 more electro-active molecules were released from the polymer beads as a function of polymer dissolution at the elevated pH. This release is reflected in the increase of the detected current. No current increase was observed at pH 5.

## Claims

1. Apparatus for the quantitative analysis of an analyte in a sample, comprising
(i) a solid phase; and
(ii) a detector,
wherein the surface of the solid phase comprises:
(a) a first position for sample application; and
(b) a second position, distant from the first position,
wherein a first molecule that binds to the analyte and is capable of releasing a detectable species is either deposited at the first position or is added to the sample prior to application to the solid phase, and
wherein a second molecule that binds to the analyte is immobilised at the second position, and
wherein an enzyme is co-locatedly immobilised with the immobilised molecule at the second position, and
wherein the detector is located in close proximity to the immobilised molecule at the second position,
wherein a substrate for the enzyme is either normally present in the sample, is added to the sample prior to application to the solid phase, or is deposited at the first position on the solid phase prior to sample application, and
wherein interaction between the enzyme and the substrate results in release of a detectable species from the first molecule.

2. Apparatus according to claim 1, further comprising means for converting the detection of said species by the detector into a value of concentration of analyte in the sample.

3. Apparatus according to any preceding claim, wherein the detector is an electrode and the detectable species is an encapsulated redox species.

4. Apparatus according to any preceding claim, wherein the enzyme is urease and the substrate is urea.

5. Apparatus according to any preceding claim, wherein the solid phase is a lateral flow membrane.

6. A method for quantitative detection of target molecules in a substrate-containing sample, comprising:
a) dissolving a first soluble labelled binding molecule that is capable of releasing a detectable species and a second soluble labelled binding molecule in the sample to form a complex;
b) applying the complex formed in step a) to a solid phase, wherein the surface comprises
i) a first position for application of the complex, and
ii) a second position, distant from the first position
wherein a molecule that binds to the second binding molecule is immobilised at the second position, wherein an enzyme is co-locatedly immobilised with the immobilised molecule at the second position, and wherein a detector is located in close proximity to the immobilised molecule at the second position; and
c) detecting the detectable species at the detector as the complex is transported to the second position,
wherein detection of said species is proportional to the target molecule content of the sample.

7. A method according to claim 6, wherein the target molecule is an antigen.

8. A method according to claim 6 or claim 7, wherein the first and/or second binding molecules are antibodies, preferably wherein the first antibody is labelled with an encapsulated redox probe, and preferably wherein the second antibody is labelled with biotin and the molecule that binds to it and is immobilised at the second position is avidin.

9. A method according to any of claims 6 to 8, wherein step (c) comprises detecting the change in current or charge passed at the detector due to the change in local pH and resultant release of the redox probe, preferably wherein the current resulting from oxidation or reduction of the redox probe is proportional to the antigen content of the sample.

10. A method according to any of claims 6 to 9, wherein the detector is an electrode preferably wherein the electrode is a screen printed electrode.

11. A method according to any of claims 6 to 10, wherein the redox probe is encapsulated in a polymer.

12. A method according to claim 11, wherein the polymer is an enteric coating which dissolves under alkaline conditions.

13. A method according to claim 11, wherein the polymer swells in acidic or alkaline conditions.

14. A method according to any of claims 6 to 13, wherein the sample is a urea-containing sample, and preferably wherein the enzyme is urease.

15. Use of apparatus according to any of claims 1 to 5 in a method according to any of claims 6 to 14.

## Patentansprüche

1. Vorrichtung zur quantitativen Analyse eines Analyten in einer Probe, beinhaltend
(i) eine feste Phase; und
(ii) einen Detektor,
wobei die Oberfläche der festen Phase Folgendes beinhaltet:
(a) eine erste Position zur Probenapplikation; und
(b) eine von der ersten Position entfernt liegende zweite Position,
wobei ein erstes Molekül, das an den Analyten bindet und in der Lage ist, eine nachweisbare Spezies freizusetzen, entweder an der ersten Position abgelagert wird oder vor der Applikation auf die feste Phase zu der Probe zugegeben wird, und
wobei ein zweites Molekül, das an den Analyten bindet, an der zweiten Position immobilisiert wird, und
wobei ein Enzym ortsgleich mit dem immobilisierten Molekül an der zweiten Position immobilisiert wird und
wobei der Detektor sich in unmittelbarer Nähe des immobilisierten Moleküls an der zweiten Position befindet,
wobei ein Substrat für das Enzym entweder normalerweise in der Probe vorliegt, vor der Applikation zur festen Phase zu der Probe zugegeben wird oder vor der Probenapplikation an der ersten Position auf der festen Phase abgelagert wird, und
wobei die Interaktion zwischen dem Enzym und dem Substrat zur Freisetzung einer nachweisbaren Spezies aus dem ersten Molekül führt.

2. Vorrichtung gemäß Anspruch 1, die ferner Mittel beinhaltet, um den Nachweis der Spezies durch den Detektor in einen Wert der Analytenkonzentration in der Probe umzuwandeln.

3. Vorrichtung gemäß einem der vorhergehenden Ansprüche, wobei der Detektor eine Elektrode ist und die nachweisbare Spezies eine verkapselte Redoxspezies ist.

4. Vorrichtung gemäß einem der vorhergehenden Ansprüche, wobei das Enzym Urease ist und das Substrat Harnstoff ist.

5. Vorrichtung gemäß einem der vorhergehenden Ansprüche, wobei die feste Phase eine laterale Flussmembran ist.

6. Ein Verfahren zum quantitativen Nachweis von Zielmolekülen in einer substrathaltigen Probe, das Folgendes beinhaltet:
(a) Auflösen eines ersten löslichen markierten Bindungsmoleküls, das in der Lage ist, eine nachweisbare Spezies freizusetzen, und eines zweiten löslichen markierten Bindungsmoleküls in der Probe, um einen Komplex zu bilden;
(b) Applizieren des in Schritt a) gebildeten Komplexes auf eine feste Phase, wobei die Oberfläche Folgendes beinhaltet:
i) eine erste Position zum Applizieren des Komplexes, und
ii) eine von der ersten Position entfernt liegende zweite Position
wobei ein Molekül, das an das zweite Bindungsmolekül bindet, an der zweiten Position immobilisiert wird, wobei ein Enzym ortsgleich mit dem immobilisierten Molekül an der zweiten Position immobilisiert wird und wobei ein Detektor sich in unmittelbarer Nähe des immobilisierten Moleküls an der zweiten Position befindet; und
(c) Nachweisen der nachweisbaren Spezies an dem Detektor, während der Komplex zu der zweiten Position transportiert wird,
wobei der Nachweis der Spezies proportional zu dem Zielmolekülgehalt der Probe ist.

7. Ein Verfahren gemäß Anspruch 6, wobei das Zielmolekül ein Antigen ist.

8. Ein Verfahren gemäß Anspruch 6 oder Anspruch 7, wobei das erste und/oder zweite Bindungsmolekül Antikörper sind, wobei der erste Antikörper vorzugsweise mit einer verkapselten Redoxprobe markiert ist und wobei der zweite Antikörper vorzugsweise mit Biotin markiert ist und das Molekül, das daran bindet und an der zweiten Position immobilisiert wird, Avidin ist.

9. Ein Verfahren gemäß einem der Ansprüche 6 bis 8, wobei Schritt (c) das Nachweisen der Änderung des Stroms oder der Ladung, die an dem Detektor passieren, aufgrund der Änderung des lokalen pH-Werts und der resultierenden Freisetzung der Redoxprobe beinhaltet, wobei der Strom, der aus Oxidation oder Reduktion der Redoxprobe resultiert, vorzugsweise proportional zu dem Antigengehalt der Probe ist.

10. Ein Verfahren gemäß einem der Ansprüche 6 bis 9, wobei der Detektor eine Elektrode ist, wobei die Elektrode vorzugsweise eine Siebdruckelektrode ist.

11. Ein Verfahren gemäß einem der Ansprüche 6 bis 10, wobei die Redoxprobe in einem Polymer verkapselt ist.

12. Ein Verfahren gemäß Anspruch 11, wobei das Polymer eine magensaftresistente Beschichtung ist, die sich unter alkalischen Bedingungen auflöst.

13. Ein Verfahren gemäß Anspruch 11, wobei das Polymer unter sauren oder alkalischen Bedingungen anschwillt.

14. Ein Verfahren gemäß einem der Ansprüche 6 bis 13, wobei die Probe eine harnstoffhaltige Probe ist und wobei das Enzym vorzugsweise Urease ist.

15. Verwendung der Vorrichtung gemäß einem der Ansprüche 1 bis 5 in einem Verfahren gemäß einem der Ansprüche 6 bis 14.

## Revendications

1. Appareil utilisé pour réaliser l'analyse quantitative d'un analyte dans un échantillon comprenant :
(i) une phase solide ; et
(ii) un détecteur,
dans lequel la surface de la phase solide comprend :
(a) une première position d'application de l'échantillon ; et
(b) une seconde position, distante de la première position,
dans laquelle une première molécule qui se lie à l'analyte et est apte à libérer une espèce détectable est soit déposée dans la première position, soit ajoutée à l'échantillon avant son application à la phase solide, et
dans laquelle la seconde molécule qui se lie à l'analyte est immobilisée dans la seconde position, et
dans laquelle l'enzyme est immobilisé en colocalisation avec la molécule immobilisée dans la seconde position, et
dans laquelle le détecteur est situé à proche proximité de la molécule immobilisée dans la seconde position,
dans laquelle un substrat pour l'enzyme est soit normalement présent dans l'échantillon, soit ajouté à l'échantillon avant son application à la phase solide, soit déposé dans la première position sur la phase solide avant l'application de l'échantillon, et
dans laquelle l'interaction entre l'enzyme et le substrat résulte en la libération d'une espèce détectable à partir de la première molécule.

2. Appareil selon la revendication 1, comprenant en outre un moyen de conversion de la détection de ladite espèce par le détecteur en une valeur de concentration de l'analyte dans l'échantillon.

3. Appareil selon l'une quelconque des revendications précédentes, dans lequel le détecteur est une électrode et l'espèce détectable est une espèce redox encapsulée.

4. Appareil selon l'une quelconque des revendications précédentes, dans lequel l'enzyme est l'uréase et le substrat est l'urée.

5. Appareil selon l'une quelconque des revendications précédentes, dans lequel la phase solide est une membrane à flux circulant de manière latérale.

6. Appareil utilisé pour réaliser une détection quantitative de molécules cibles dans un échantillon contenant un substrat, comprenant les étapes consistant à :
a) dissoudre une première molécule de liaison marquée soluble apte à libérer une espèce détectable et une seconde molécule de liaison marquée soluble dans l'échantillon pour former un complexe ;
b) appliquer le complexe formé à l'étape a) à une phase solide, la surface comprenant
i) une première position pour l'application du complexe, et
ii) une seconde position, distante de la première position
dans laquelle une molécule qui se lie à la seconde molécule de liaison est immobilisée dans la seconde position, dans laquelle un enzyme est immobilisé en colocalisation avec la molécule immobilisée dans la seconde position et dans laquelle un détecteur est placé à proche proximité de la molécule immobilisée dans la seconde position ; et
c) détecter l'espèce détectable au niveau du détecteur alors que le complexe est transporté dans la seconde position,
la détection de ladite espèce étant proportionnelle au contenu de la molécule cible de l'échantillon.

7. Procédé selon la revendication 6, dans lequel la molécule cible est un antigène.

8. Procédé selon la revendication 6 ou la revendication 7, dans lequel les première et/ou seconde molécules sont des anticorps, de préférence dans lequel le premier anticorps est marqué avec une sonde redox encapsulée et de préférence dans lequel le second anticorps est marquée de biotine et la molécule qui s'y lie et qui est immobilisée dans la seconde position est l'avidine.

9. Procédé selon l'une quelconque des revendications 6 à 8, dans lequel l'étape (c) comprend la détection du changement de courant ou de charge passé au niveau du détecteur en raison d'un changement du pH local et de la libération résultante de la sonde de redox, de préférence dans lequel le courant résultant de l'oxydation ou de la réduction de la sonde de redox est proportionnel à la teneur en antigène de l'échantillon.

10. Procédé selon l'une quelconques des revendications 6 à 9, dans lequel le détecteur est une électrode, de préférence dans lequel l'électrode est une électrode imprimée par sérigraphie.

11. Procédé selon l'une quelconque des revendications 6 à 10, dans lequel la sonde de redox est encapsulée dans un polymère.

12. Procédé selon la revendication 11, dans lequel le polymère est un enrobage entérosoluble qui se dissous sous des conditions alcalines.

13. Procédé selon la revendication 11, dans lequel le polymère gonfle dans des conditions acides ou alcalines.

14. Procédé selon l'une quelconque des revendications 6 à 13, dans lequel l'échantillon est un échantillon contenant de l'urée et de préférence dans lequel l'enzyme est l'uréase.

15. Utilisation de l'appareil selon l'une quelconque des revendications 1 à 5 dans un procédé selon l'une quelconque des revendications 6 à 14.
